(19) Europäisches Patentamt
European Patent Office
Office européen des brevets

(11) Veröffentlichungsnummer: **0 112 987**
**B1**

# (12) EUROPÄISCHE PATENTSCHRIFT

(45) Veröffentlichungstag der Patentschrift: **01.04.87**

(51) Int. Cl.⁴: **C 07 D 409/14**, A 61 K 31/50, A 61 K 31/415, A 61 K 31/38

(21) Anmeldenummer: **83110493.0**

(22) Anmeldetag: **21.10.83**

(54) **Imidazolylalkylthienyl-tetrahydropyridazine und Verfahren zu ihrer Herstellung.**

(30) Priorität: **06.11.82 DE 3241102**

(43) Veröffentlichungstag der Anmeldung: **11.07.84 Patentblatt 84/28**

(45) Bekanntmachung des Hinweises auf die Patenterteilung: **01.04.87 Patentblatt 87/14**

(84) Benannte Vertragsstaaten: **AT BE CH DE FR GB IT LI LU NL SE**

(56) Entgegenhaltungen:
**EP - A - 0 071 059**
**EP - A - 0 075 436**
**US - A - 4 504 479**

**Die Akte enthält technische Angaben, die nach dem Eingang der Anmeldung eingereicht wurden und die nicht in dieser Patentschrift enthalten sind.**

(73) Patentinhaber: **A. Nattermann & Cie. GmbH, Nattermannallee 1, D-5000 Köln 30 (DE)**

(72) Erfinder: **Hilboll, Gerd, Dr., Dehmelstrasse 36, D-5000 Köln 30 (DE)**
Erfinder: **Prop, Gerrit, Dr., Mohnblumenweg 25, D-5024 Pulheim (DE)**
Erfinder: **Borbe, Harald, Dr., Lübecker Strasse 5, D-5000 Köln 40 (DE)**
Erfinder: **Löhr, Josef Peter, Dr., Henkenheide 55, D-4010 Hilden (DE)**
Erfinder: **Doppelfeld, Ille-Stephanie, Im Bruchfeldchen 35, D-5010 Giessen (DE)**

(74) Vertreter: **Redies, Bernd, Dr. rer. nat., COHAUSZ & FLORACK Patentanwaltsbüro Schumannstrasse 97 Postfach 14 01 47, D-4000 Düsseldorf 1 (DE)**

EP 0 112 987 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft neue Imidazolylalkylthienyl-tetrahydropyridazine und deren physiologisch verträgliche Säureadditionssalze, Verfahren zu ihrer Herstellung sowie ihre Verwendung als Wirkstoffe in Arzneimitteln.

Die erfindungsgemässen Imidazolylalkylthienyl-tetrahydropyridazine entsprechen der allgemeinen Formel I

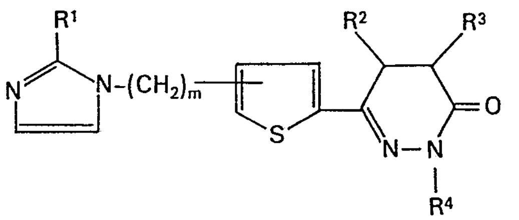

(I)

worin $R^1$, $R^2$, $R^3$ und $R^4$ gleich oder verschieden sein können und unabhängig von einander ein Wasserstoffatom, einen $C_{1-4}$-Niederalkylrest oder einen Phenylrest bedeuten, während m für Null oder eine ganze Zahl von 1–8 steht; ausgenommen sind dabei Verbindungen mit 2,5-disubstituiertem Thiophenrest, bei denen $R^1$, $R^2$, $R^3$, $R^4$ gleichzeitig Wasserstoff bedeuten und m ungleich Null ist.

Besonders bevorzugt sind dabei Verbindungen mit 2,5- bzw. 2,4-disubstituierten Thiopehnresten, bei denen m Null ist und der Pyridazinring in 5-Stellung eine Methylgruppe trägt.

Verbindungen gemäss der Erfindung sind beispielsweise:

6-[5-(1-Imidazolyl-methyl)-thien-2-yl]-3-oxo-2-phenyl-2,3,4,5-tetrahydro-pyridazin.
5-[4-(1-Imidazolyl-methyl)-thien-2-yl]-3-oxo-2,3,4,5-tetrahydro-pyridazin.
6-[5-(1-Imidazolyl)-thien-2-yl]-5-methyl-3-oxo-2,3,4,5-tetrahydro-pyridazin.
6-[5-(1-Imidazolyl)-thien-2-yl]-2,5-dimethyl-3-oxo-2,3,4,5-tetrahydro-pyridazin.
6-[5-(2-Methyl-1-imidazolyl)-thien-2-yl]-5-methyl-3-oxo-2,3,4,5-tetrahydro-pyridazin.
6-[4-(1-Imidazolyl)-thien-2-yl]-5-methyl-3-oxo-2,3,4,5-tetrahydro-pyridazin.

Darin enthalten sind auch die Säureadditionssalze von Verbindungen der allgemeinen Formel I. Säureadditionssalze sind insbesondere pharmazeutisch verwendbare, z.B. solche mit Chlorwasserstoffsäure, Bromwasserstoffsäure, Schwefelsäure oder Phosphorsäure, oder mit organischen Säuren, wie entsprechende Carbonsäuren, z.B. Essigsäure, Propionsäure, Oxalsäure, Malonsäure, Glykolsäure, Bernsteinsäure, Maleinsäure, Fumarsäure, Äpfelsäure, Citronensäure oder Weinsäure.

Die Verbindungen der allgemeinen Formel I besitzen ein Chiralitätszentrum an den Positionen 4 bzw. 5 des Pyridazinringes, wenn die Substituenten $R^2$ und/oder $R^3$ ungleich Wasserstoff sind und können somit als Racemate oder in Form der Enantiomeren vorliegen. Falls eine Trennung der Racemate erwünscht ist, wird diese zweckmässigerweise nach an sich bekannten Verfahren mit einer optisch aktiven Säure, wie z.B. Dibenzoylweinsäure oder Campher-10-sulfonsäure über die Bildung diastereomerer Salze oder durch Chromatographie an optisch aktivem Säulenmaterial durchgeführt.

Die erfindungsgemässen Verbindungen der allgemeinen Formel I besitzen wertvolle pharmakologische Eigenschaften. Insbesondere zeichnen sie sich durch starke blutdrucksenkende sowie antithrombotische Wirkung aus. Ausserdem beeinflussen sie den Arachidonsäure-Metabolismus und zeigen eine antagonistische Wirkung bezüglich einiger durch PAF (Platelet Aktivating Factor) geregelter physiologischer Vorgänge. Daneben zeigen die Verbindungen der allgemeinen Formel I eine günstige Beeinflussung asthmatischer Beschwerden sowie antirheumatische und antiatherosklerotische Aktivität. Sie lassen sich daher zur Behandlung von cardiovaskulären und thromboembolischen Erkrankungen, insbesondere am Menschen, nutzen.

Falls erwünscht, können die Verbindungen der allgemeinen Formel I mit Diuretika und Betablokkern kombiniert werden.

Die Darstellung der erfindungsgemässen Substanzen der allgemeinen Formel I erfolgt durch Umsetzung der 4-[(1-Imidazolylalkyl)-thien-2-yl]-4-oxo-buttersäuren oder deren Alkylestern der allgemeinen Formel II, in der $R^1$, $R^2$, $R^3$ und m die in der allgemeinen Formel I angegebene Bedeutung haben und $R^5$ Wasserstoff der $C_{1-6}$-Alkyl bedeutet, mit einer Hydrazinverbindung der allgemeinen Formel III, in der $R^4$ die in der allgemeinen Formel I angegebene Bedeutung hat, deren Hydrat oder Salz, wie Hydrochlorid, Hydrogensulfat, Sulfat o.ä., in wässrigen, wässrig-alkoholischen, alkoholischen Medien oder in unter den gewählten Bedingungen indifferenten Lösungsmitteln, wie z.B. Dioxan, Toluol, Dimethylformamid, oder deren Mischungen mit Wasser oder Alkohol bei Temperaturen von 30–150°C, gegenbenenfalls unter Zuhilfenahme eines der für Aminolysen und Kondensationsreaktionen üblichen Katalysatoren, wie z.B. Bariumoxid, vorzugsweise bei 80–100°C in Ethanol oder Wasser.

Die Umsetzung wird durch folgendes Reaktionsschema veranschaulicht:

(II)

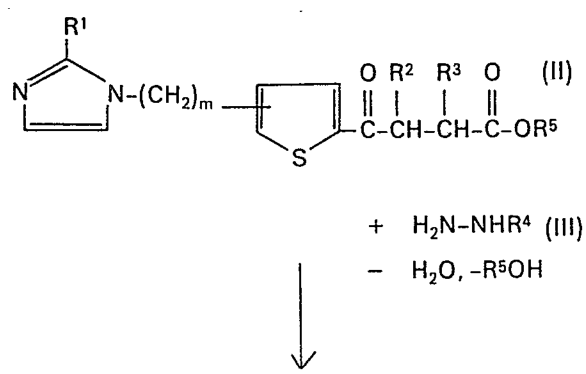

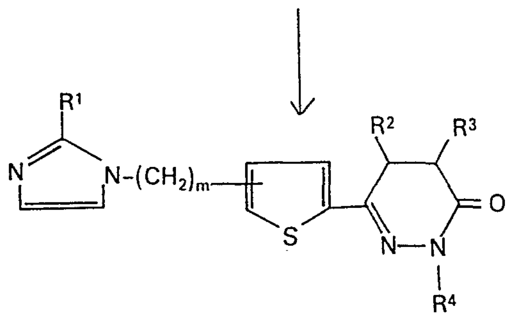

(I)

Als Ausgangsverbindungen der allgemeinen Formel II kommen insbesondere in Frage:

4-[5-(1-Imidazolyl-methyl)-thien-2-yl]-4-oxo-buttersäure sowie deren $C_{1-6}$-Alkylester,
4-[5-(2-Methyl-1-imidazolyl-methyl)-thien-2-yl]-4-oxo-buttersäure sowie deren $C_{1-6}$-Alkylester,
4-[5-(2-Ethyl-1-imidazolyl-methyl)-thien-2-yl]-4-oxo-buttersäure sowie deren $C_{1-6}$-Alkylester,
4-[5-(2-Propyl-1-imidazolyl-methyl)-thien-2-yl]-4-oxo-buttersäure sowie deren $C_{1-6}$-Alkylester,
4-{5-[2-(2-Methyl-1-imidazolyl)-ethyl]-thien-2-yl}-4-oxo-buttersäure sowie deren $C_{1-6}$-Alkylester,
4-{5-[3-(2-Methyl-1-imidazolyl)-propyl]-thien-2-yl}-4-oxo-buttersäure sowie deren $C_{1-6}$-Alkylester,
4-{5-[4-(2-Methyl-1-imidazolyl)-butyl]-thien-2-yl}-4-oxo-buttersäure sowie deren $C_{1-6}$-Alkylester,
4-{5-[5-(2-Methyl-1-imidazolyl)-pentyl]-thien-2-yl}-4-oxo-buttersäure sowie deren $C_{1-6}$-Alkylester,
4-{5-[6-(2-Methyl-1-imidazolyl)-hexyl]-thien-2-yl}-4-oxo-buttersäure sowie deren $C_{1-6}$-Alkylester,
4-{5-[7-(2-Methyl-1-imidazolyl)-heptyl]-thien-2-yl}-4-oxo-buttersäure sowie deren $C_{1-6}$-Alkylester,
4-{5-[8-(2-Methyl-1-imidazolyl)-octyl]-thien-2-yl}-4-oxo-buttersäure sowie deren $C_{1-6}$-Alkylester,
4-[4-(1-Imidazolyl-methyl)-thien-2-yl]-4-oxo-buttersäure sowie deren $C_{1-6}$-Alkylester,
4-[4-(2-Methyl-1-imidazolyl-methyl)-thien-2-yl]-4-oxo-buttersäure sowie deren $C_{1-6}$-Alkylester,
4-[4-(2-Ethyl-1-imidazolyl-methyl)-thien-2-yl]-4-oxo-buttersäure sowie deren $C_{1-6}$-Alkylester,
4-[4-(2-Propyl-1-imidazolyl)-methyl-thien-2-yl]-4-oxo-buttersäure sowie deren $C_{1-6}$-Alkylester,
4-[5-(1-Imidazolyl)-thien-2-yl]-4-oxo-buttersäure sowie deren $C_{1-6}$-Alkylester,
4-[5-(1-Imidazolyl)-thien-2-yl]-2-methyl-4-oxo-buttersäure sowie deren $C_{1-6}$-Alkylester,
4-[5-(1-Imidazolyl)-thien-2-yl]-3-methyl-4-oxo-buttersäure sowie deren $C_{1-6}$-Alkylester,
4-[5-(1-Imidazolyl)-thien-2-yl]-2-ethyl-4-oxo-buttersäure sowie deren $C_{1-6}$-Alkylester,
4-[5-(1-Imidazolyl)-thien-2-yl]-3-ethyl-4-oxo-buttersäure sowie deren $C_{1-6}$-Alkylester,
4-[5-(1-Imidazolyl)-thien-2-yl]-4-oxo-2-propyl-buttersäure sowie deren $C_{1-6}$-Alkylester,
4-[5-(1-Imidazolyl)-thien-2-yl]-4-oxo-3-propyl-buttersäure sowie deren $C_{1-6}$-Alkylester,
4-[5-(1-Imidazolyl)-thien-2-yl]-3-isopropyl-4-oxo-buttersäure sowie deren $C_{1-6}$-Alkylester,
4-[5-(1-Imidazolyl)-thien-2-yl]-4-oxo-2-phenyl-buttersäure sowie deren $C_{1-6}$-Alkylester,
4-[5-(1-Imidazolyl)-thien-2-yl]-4-oxo-3-phenyl-buttersäure sowie deren $C_{1-6}$-Alkylester,
4-[5-(2-Methyl-1-imidazolyl)-thien-2-yl]-4-oxo-buttersäure sowie deren $C_{1-6}$-Alkylester,
4-[5-(2-Methyl-1-imidazolyl)-thien-2-yl]-2-methyl-4-oxo-buttersäure sowie deren $C_{1-6}$-Alkylester,
4-[5-(2-Methyl-1-imidazolyl)-thien-2-yl]-3-methyl-4-oxo-buttersäure sowie deren $C_{1-6}$-Alkylester,
4-[5-(2-Ethyl-1-imidazolyl)-thien-2-yl]-4-oxo-buttersäure sowie deren $C_{1-6}$-Alkylester,
4-[5-(2-Ethyl-1-imidazolyl)-thien-2-yl]-2-methyl-4-oxo-buttersäure sowie deren $C_{1-6}$-Alkylester,
4-[5-(2-Ethyl-1-imidazolyl)-thien-2-yl]-3-methyl-4-oxo-buttersäure sowie deren $C_{1-6}$-Alkylester,
4-[5-(2-Propyl-1-imidazolyl)-thien-2-yl]-4-oxo-buttersäure sowie deren $C_{1-6}$-Alkylester,
4-[5-(2-Propyl-1-imidazolyl)-thien-2-yl]-3-methyl-4-oxo-buttersäure sowie deren $C_{1-6}$-Alkylester,
4-[5-(2-Phenyl-1-imidazolyl)-thien-2-yl]-3-methyl-4-oxo-buttersäure sowie deren $C_{1-6}$-Alkylester,
4-[4-(1-Imidazolyl)-thien-2-yl]-4-oxo-buttersäure sowie deren $C_{1-6}$-Alkylester,
4-[4-(1-Imidazolyl)-thien-2-yl]-2-methyl-4-oxo-buttersäure sowie deren $C_{1-6}$-Alkylester,
4-[4-(1-Imidazolyl)-thien-2-yl]-3-methyl-4-oxo-buttersäure sowie deren $C_{1-6}$-Alkylester,
4-[4-(2-Methyl-1-imidazolyl)-thien-2-yl]-3-methyl-4-oxo-buttersäure sowie deren $C_{1-6}$-Alkylester.

Als Ausgangsverbindungen der allgemeinen Formel III kommen insbesondere in Frage:

Hydrazin, Methylhydrazin, Ethylhydrazin, Propylhydrazin, Phenylhydrazin sowie deren Hydrate oder Salze, wie z.B. Hydrochloride, Hydrogensulfate, Sulfate u.a.

Die Darstellung der Ausgangsverbindungen der allgemeinen Formel II erfolgt nach an sich bekannten Verfahren:

a) Ausgangsverbindungen der allgemeinen Formel II mit m ungleich Null: 1-(ω-Thienylalkyl)-imidazole werden durch Alkylierung von Imidazolen mit ω-Halogenalkyl-thiophenen ggfs. unter Zusatz eines organischen Lösungsmittels, wie z.B. Dimethylformamid, unter möglicher Verwendung einer Hilfsbase, wie. z.B. Natriumhydrid, hergestellt (DT-OS 2 933 649).

Die 1-(ω-Thienylalkyl)-imidazole werden nach bekannten Verfahren (Houben-Weyl, Methoden der organischen Chemie, Bd. 7/2a, S. 257) mit Bernsteinsäurealkylesterchlorid unter Zusatz eines organischen Lösungsmittels, wie z.B. 1,2-Dichlorethan, Nitrobenzol, Schwefelkohlenstoff, unter Verwendung eines Friedel-Crafts-Katalysators, wie z.B. Aluminiumchlorid, zu den

4-[ω-(1-Imidazolyl)-alkyl-thien-2-yl]-4-oxo-buttersäurealkylestern der allgemeinen Formel II umgesetzt, die nach bekannten Verfahren zu den entsprechenden 4-[ω(1-Imidazolyl)-alkyl-thien-2-yl]-4-oxo-buttersäuren hydrolysiert werden.

Die Umsetzungen verlaufen nach folgendem Formelschema:

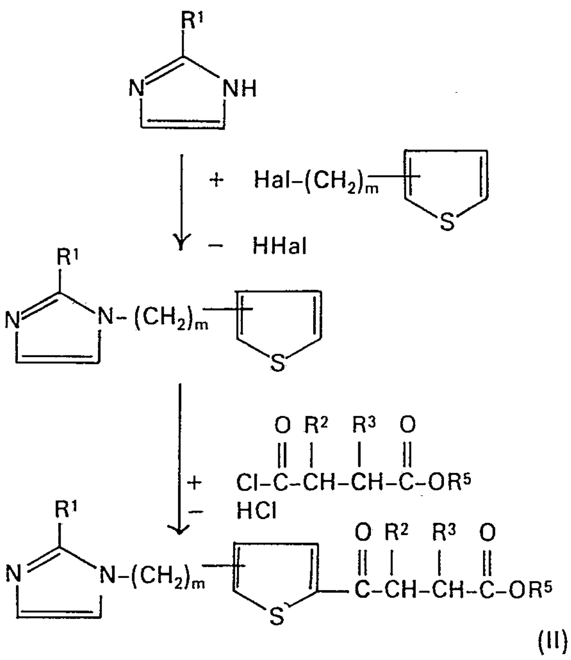


(II)

b) Ausgangsverbindungen der allgemeinen Formel II mit m = 0. Bromthiophen-2-aldehyde werden mit Imidazolen durch Erhitzen mit Kaliumcarbonat in Pyridin unter Kupferoxid-Katalyse [analog J.B. Poly u.a. J. Chem. Soc. C (1970), 85 ff] oder durch Erhitzen mit Kaliumcarbonat in Nitrobenzol unter Kupferbromid-Katalyse [analog L.M. Sitkina, A.M. Simonov, C.A. 65 (1966), 13686 e] zu den 1-Imidazolyl-thiophen-2-aldehyden umgesetzt. Diese werden unter dem katalytischen Einfluss von Natriumcyanid [analog H. Stetter, Angew. Chem. 88 (1976), 695–736] mit 2-Alkensäurenitrilen zu den entsprechenden 4-(1-Imidazolyl-thien-2-yl)-4-oxo-buttersäurenitrilen umgesetzt, die durch saure Hydrolyse in die 4-(1-Imidazolyl-thien-2-yl]-4-oxo-buttersäuren der allgemeinen Formel II umgewandelt werden.

Die Umsetzungen verlaufen nach folgendem Reaktionsschema:

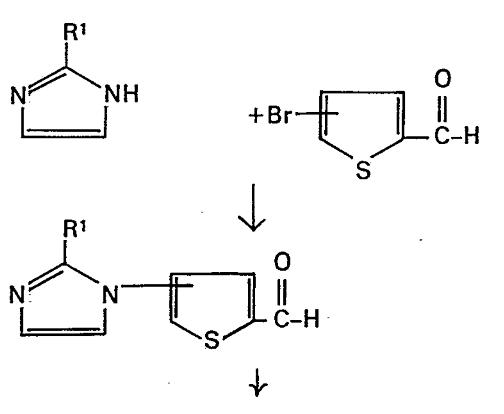

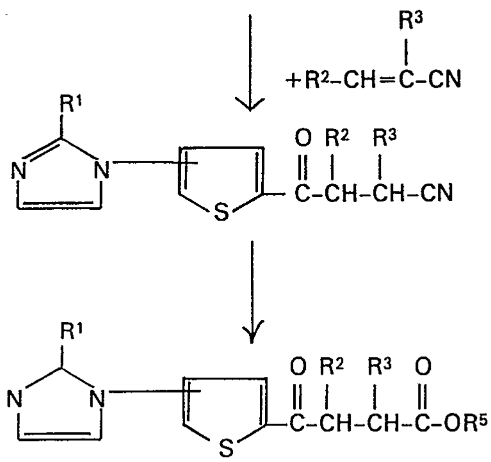

(II)

Die Säureadditionssalze von Verbindungen der allgemeinen Formel I mit anorganischen oder organischen Säuren lassen sich durch Mischen der zugrundeliegenden Imidazolylverbindungen mit den entsprechenden Säuren in wässrigen, wässrig-organischen (z.B. Alkohol-Wasser) oder organischen Medien, wie z.B. Alkoholen, Alkohol-Ether-Mischungen oder Ether-Petrolether-Mischungen bei Temperaturen zwischen 0 und 100°C herstellen.

Die Erfindung betrifft ebenfalls pharmazeutische Präparate, welche Verbindungen der allgemeinen Formel I oder pharmazeutisch verwendbare Säureadditionssalze dieser Verbindungen enthalten. Bei den erfindungsgemässen pharmazeutischen Präparaten handelt es sich um solche zur enteralen wie oralen oder rektalen sowie parenteralen Verabreichung, welche die pharmazeutischen Wirkstoffe allein oder zusammen mit einem üblichen, pharmazeutisch anwendbaren Trägermaterial enthalten. Vorteilhafterweise liegt die pharmazeutische Zubereitung des Wirkstoffes in Form von Einzeldosen vor, die auf die gewünschte Verabreichung abgestimmt sind, wie z.B. Tabletten, Dragées, Kapseln, Suppositorien, Granulate, Lösungen, Emulsionen oder Suspensionen. Die Dosierung der Verbindungen liegt üblicherweise zwischen 1–500 mg pro Dosis, vorzugsweise zwischen 5–150 mg je Dosis und kann ein- oder mehrmals, bevorzugt zwei- bis dreimal täglich, verabreicht werden.

Die Herstellung der erfindungsgemässen Verbindungen wird durch die folgenden Beispiele näher erläutert. Die angegebenen Schmelzpunkte wurden mit einem Büchi 510-Schmelzpunktbestimmungsapparat gemessen und sind mit °C angegeben und nicht korrigiert. Die IR-Spektren wurden mit den Geräten Perkin Elmer 257 bzw. Nicolet NIC-3600 und die Massenspektren mit dem Gerät Varian MAT-311-A (70 eV) aufgenommen.

Beispiel 1

6-[5-(2-Methyl-1-imidazolyl-methyl)-thien-2-yl]-3-oxo-2,3,4,5-tetrahydro-pyridazin.

Eine Mischung aus 3 g 4-[5-(2-Methyl-1-imidazolyl-methyl)-thien-2-yl]-4-oxo-buttersäure, 0,54 ml Hydrazinhydrat und 50 ml Wasser wird 2

Stunden auf 100°C erhitzt. Nach Abkühlen wird der ausgefallene Feststoff abgesaugt, mit Wasser gewaschen und getrocknet.

Ausbeute: 1,4 g Schmelzpunkt: 133–135°C
IR (in KBr): 1665, 1605 $cm^{-1}$
MS [m/e]: 274 ($M^+$, 13%), 193 (100%), 151 (21%), 135 (10%), 122 (45%).

Beispiel 2

6-[5-(1-Imidazolyl-methyl)-thien-2-yl]-2-methyl-3-oxo-2,3,4,5-tetrahydro-pyridazin.

Eine Mischung aus 8,7 g 4-[5-(1-Imidazolyl-methyl)-thien-2-yl]-4-oxo-buttersäure, 2,9 ml Methylhydrazin und 100 ml Wasser wird 2 Stunden auf 90°C erhitzt. Nach Abkühlen wird der ausgefallene Feststoff abgesaugt, in Chloroform gelöst, die Chloroformphase mit verdünnter Natronlauge und mit Wasser gewaschen, über Natriumsulfat getrocknet und eingeengt. Der Rückstand wird säulenchromatographisch (Kieselgel//Chloroform) gereinigt und anschliessend aus Ethanol umkristallisiert.

Ausbeute: 1,6 g Schmelzpunkt: 121°C
IR (in KBr): 1637 $cm^{-1}$
MS [m/e]: 274 ($M^+$, 39%), 207 (100%), 165 (6%), 149 (13%), 137 (6%), 122 (13%).

Beispiel 3

6-[5-(1-Imidazolyl-methyl)-thien-2-yl]-3-oxo-2-phenyl-2,3,4,5-tetrahydro-pyridazin.

Eine Mischung aus 2,64 g 4-[5-(1-Imidazolyl-methyl)-thien-2-yl]-4-oxo-buttersäure, 1,12 g Phenylhydrazin, 40 ml Wasser und 40 ml Ethanol wird 10 Stunden unter Rückfluss gerührt. Nach Abkühlen wird eingeengt, der Rückstand mit Chloroform/verdünnter Natronlauge geschüttelt, die Chloroformphase mit Wasser gewaschen, getrocknet und eingeengt. Der Rückstand wird aus Diisopropylether/Essigsäureethylester umkristallisiert.

Ausbeute: 160 mg Schmelzpunkt: 178°C
IR (in KBr): 1628 $cm^{-1}$
MS [m/e]: 336 ($M^+$, 46%), 269 (73%), 122 (17%), 105 (74%), 77 (100%).

Beispiel 4

6-[4-(1-Imidazolyl-methyl)-thien-2-yl]-3-oxo-2,3,4,5-tetrahydro-pyridazin.

Eine Mischung aus 5,6 g 4-[4-(1-Imidazolyl-methyl)-thien-2-yl]-4-oxo-buttersäure, 1,1 g Hydrazinhydrat und 50 ml Wasser wird 2 Stunden bei 90°C gerührt. Nach Abkühlen wird der ausgefallene Feststoff abgesaugt, mit Wasser gewaschen und getrocknet.

Ausbeute: 5,2 g Schmelzpunkt: 178–179°C
IR (in KBr): 1670 $cm^{-1}$
MS [m/e]: 260 ($M^+$, 47%), 193 (100%), 131 (5%), 122 (9%).

Analog den Beispielen 1–4 lassen sich herstellen:

6-{5-[2-(2-Methyl-1-imidazolyl)-ethyl]-thien-2-yl}-3-oxo-2,3,4,5-tetrahydro-pyridazin,
6-{5-[3-(2-Methyl-1-imidazolyl)-propyl]-thien-2-yl}-3-oxo-2,3,4,5-tetrahydro-pyridazin,
6-{5-[4-(2-Methyl-1-imidazolyl)-butyl]-thien-2-yl}-3-oxo-2,3,4,5-tetrahydro-pyridazin,
6-{5-[5-(2-Methyl-1-imidazolyl)-pentyl]-thien-2-yl}-3-oxo-2,3,4,5-tetrahydro-pyridazin,
6-{5-[6-(2-Methyl-1-imidazolyl)-hexyl]-thien-2-yl}-3-oxo-2,3,4,5-tetrahydro-pyridazin,
6-{5-[7-(2-Methyl-1-imidazolyl)-heptyl]-thien-2-yl}-3-oxo-2,3,4,5-tetrahydro-pyridazin,
6-{5-[8-(2-Methyl-1-imidazolyl)-octyl]-thien-2-yl}-3-oxo-2,3,4,5-tetrahydro-pyridazin,
6-[5-(2-Ethyl-1-imidazolyl-methyl)-thien-2-yl]-3-oxo-2,3,4,5-tetrahydro-pyridazin,
6-[5-(2-Propyl-1-imidazolyl-methyl)-thien-2-yl]-3-oxo-2,3,4,5-tetrahydro-pyridazin,
6-[5-(1-Imidazolyl-methyl)-thien-2-yl]-2-ethyl-3-oxo-2,3,4,5-tetrahydro-pyridazin,
6-[5-(1-Imidazolyl-methyl)-thien-2-yl]-3-oxo-2-propyl-2,3,4,5-tetrahydro-pyridazin.

Beispiel 5

6-[5-(1-Imidazolyl)-thien-2-yl]-5-methyl-3-oxo-2,3,4,5-tetrahydro-pyridazin.

a) 4-[5-(1-Imidazolyl)-thien-2-yl]-3-methyl-4-oxo-buttersäurenitril.

Eine Mischung aus 14,7 g 5-(1-Imidazolyl)-thiophen-2-aldehyd [hergestellt analog J.B. Polya u.a., Chem. Soc. C (1970), 85 ff, Schmp. 105–107°C], 5,5 g 2-Butensäurenitril, 0,81 g Natriumcyanid und 200 ml Dimethylformamid wird 16 Stunden bei 25°C unter Stickstoffatmosphäre gerührt. Nach Zugabe von Wasser wird mit Chloroform extrahiert, die Chloroformphase mit Wasser gewaschen, getrocknet, eingeengt und der Rückstand durch Säulenchromatographie (Kieselgel//Chloroform/Methanol) gereinigt.

Ausbeute: 9,4 g Schmelzpunkt: 112–114°C
IR (in KBr): 2245, 1648 $cm^{-1}$
MS [m/e]: 245 ($M^+$, 37%), 177 (100%), 149 (17%), 122 (8%), 105 (14%).

b) 4-[5-(1-Imidazolyl)-thien-2-yl]-3-methyl-4-oxo-buttersäure.

6,7 g Oxobuttersäurenitril werden in 50 ml 18%iger Salzsäure 3 Stunden unter Rückfluss erhitzt. Nach Abkühlen wird mit Natronlauge auf pH 9 eingestellt und mit Chloroform gewaschen. Anschliessend wird auf pH 6,5 eingestellt. Der ausgefallene Feststoff wird abgesaugt und getrocknet. Das Filtrat wird auf ein Restvolumen von 80 ml eingeengt und ohne weitere Reinigung in die nächste Stufe eingesetzt.

Ausbeute: 0,45 g Schmelzpunkt: 180–182°C
IR (in KBr): 1721, 1638 $cm^{-1}$
MS [m/e]: 264 ($M^+$, 30%), 177 (100%), 149 (17%), 122 (8%), 105 (16%).

c) 6-[5-(1-Imidazolyl)-thien-2-yl]-5-methyl-3-oxo-2,3,4,5-tetrahydro-pyridazin.

Die wässrige Lösung 5 b) wird mit 3 ml Hydrazinhydrat 2 Stunden bei 90°C gerührt. Der beim Abkühlen ausgefallene Feststoff wird aus wenig Chloroform umkristallisiert.

Ausbeute: 3,3 g Schmelzpunkt: 239–241°C
IR (in KBr): 1677 $cm^{-1}$
MS [m/e]: 260 ($M^+$, 100%), 245 (12%), 203 (16%), 189 (14%), 175 (6%), 149 (6%).

Beispiel 6

6-[5-(1-Imidazolyl)-thien-2-yl]-2,5-dimethyl-3-oxo-2,3,4,5-tetrahydro-pyridazin.

Eine Mischung aus 400 mg Oxobuttersäure aus Beispiel 5b), 86 mg Methylhydrazin und 10 ml Wasser wird 2 Stunden bei 90°C gerührt. Nach Abkühlen wird mit Chloroform extrahiert, die Chloroformphase eingeengt und der Rückstand durch Dünnschichtchromatographie (Kieselgel-Fertigplatte 60 F 254, Chloroform/Methanol 90/10) gereinigt.

Ausbeute: 14 mg Schmelzpunkt: 134–136°C

Rf-Wert: 0,53

MS [m/e]: 274 ($M^+$, 100%), 259 (4%), 203 (16%), 189 (20%), 163 (6%), 71 (21%).

Beispiel 7

6-[5-(2-Methyl-1-imidazolyl)-thien-2-yl]-5-methyl-3-oxo-2,3,4,5-tetrahydro-pyridazin.

Eine Mischung aus 1 g 5-(2-Methyl-1-imidazolyl)-thiophen-2-aldehyd (Schmp. 86–88°C), 700 mg 2-Butensäurenitril, 41 mg Natriumcyanid und 25 ml Dimethylformamid wird 3 Tage unter Stickstoffatmosphäre gerührt. Nach Zugabe von Wasser wird mit Chloroform extrahiert, die organische Phase mit Wasser gewaschen, getrocknet und eingeengt. Der verbleibende Rückstand (300 mg) wird mit 20 ml 18%iger Salzsäure 2 Stunden auf Rückflusstemperatur gehalten. Nach Abkühlen wird mit Natronlauge auf pH 9 eingestellt, mit Chloroform extrahiert und die Wasserphase mit verdünnter Salzsäure auf pH 7 eingestellt. Nach Einengen auf ein Restvolumen von 50 ml werden 0,5 ml Hydrazinhydrat zugegeben und die Mischung 18 Stunden bei 90°C gerührt. Nach Abkühlen wird mit Chloroform extrahiert, eingeengt und der Rückstand chromatographisch (Kieselgel-Fertigplatte 60 F 254, Chloroform/Methanol 90/10) gereinigt.

Ausbeute: 30 mg Schmelzpunkt: 195–197°C

Rf-Wert: 0,5

MS [m/e]: 274 ($M^+$, 100%), 259 (9%), 217 (10%), 206 (16%).

Beispiel 8

6-[4-(1-Imidazolyl)-thien-2-yl]-5-methyl-3-oxo-2,3,4,5-tetrahydro-pyridazin.

a) 4-[4-(1-Imidazolyl)-thien-2-yl]-3-methyl-4-oxo-buttersäurenitril.

Analog Beispiel 5a) wird die Reaktion durchgeführt mit 6 g 4-(1-Imidazolyl)-thiophen-2-aldehyd (Schmp. 127–130°C), 2,3 g 2-Butensäurenitril, 0,45 g Natriumcyanid und 50 ml Dimethylformamid.

Ausbeute: 3,9 g Schmelzpunkt: 113°C

IR (in KBr): 2252, 1654 $cm^{-1}$

MS [m/e]: 245 ($M^+$, 38%), 177 (100%), 149 (11%), 122 (5%), 105 (12%).

b) 4-[4-(1-Imidazolyl)-thien-2-yl]-3-methyl-4-oxo-buttersäure. 67 g Oxobuttersäurenitril aus Beispiel 8a) werden in 600 ml 16%iger Salzsäure 5 Stunden unter Rückfluss erhitzt. Nach Abkühlen wird mit 6-N-Natronlauge auf pH 6,4 eingestellt, der ausgefallene Feststoff wird abgesaugt, mit Wasser gewaschen und getrocknet.

Ausbeute: 63,6 g Schmelzpunkt: 230–231°C

IR (in KBr): 1718, 1668 $cm^{-1}$

MS [m/e]: 264 ($M^+$, 39%), 195 (12%), 177 (100%), 149 (13%), 122 (5%), 105 (11%).

c) 6-[4-(1-Imidazolyl)-thien-2-yl]-5-methyl-3-oxo-2,3,4,5-tetrahydro-pyridazin.

26,4 g Oxobuttersäure aus Beispiel 8b) werden mit 6 g Hydrazinhydrat in 200 ml Wasser 2 Stunden unter Rückfluss gerührt. Das in der Hitze ausgefallene Produkt wird heiss abgesaugt, mit Wasser gewaschen und getrocknet.

Ausbeute: 17,7 g Schmelzpunkt: 225°C

IR (in KBr): 1671 $cm^{-1}$

MS [m/e]: 260 ($M^+$, 100%), 245 (10%), 217 (3%), 203 (19%), 189 (16%), 176 (7%), 148 (7%), 121 (5%).

Analog den Beispielen 5–8 werden hergestellt:

6-[5-(1-Imidazolyl)-thien-2-yl]-3-oxo-2,3,4,5-tetrahydro-pyridazin,
6-[5-(1-Imidazolyl)-thien-2-yl]-2-methyl-3-oxo-2,3,4,5-tetrahydro-pyridazin,
6-[5-(1-Imidazolyl)-thien-2-yl]-4-methyl-3-oxo-2,3,4,5-tetrahydro-pyridazin,
6-[5-(1-Imidazolyl)-thien-2-yl]-5-ethyl-3-oxo-2,3,4,5-tetrahydro-pyridazin,
6-[5-(2-Methyl-1-imidazolyl)-thien-2-yl]-3-oxo-2,3,4,5-tetrahydro-pyridazin,
6-[5-(2-Ethyl-1-imidazolyl)-thien-2-yl]-5-methyl-3-oxo-2,3,4,5-tetrahydro-pyridazin,
6-[5-(2-Phenyl-1-imidazolyl)-thien-2-yl]-5-methyl-3-oxo-2,3,4,5-tetrahydro-pyridazin,
6-[4-(1-Imidazolyl)-thien-2-yl]-3-oxo-2,3,4,5-tetrahydro-pyridazin,
6-[4-(1-Imidazolyl)-thien-2-yl]-5-ethyl-3-oxo-2,3,4,5-tetrahydro-pyridazin,
6-[4-(2-Methyl-1-imidazolyl)-thien-2-yl]-5-methyl-3-oxo-2,3,4,5-tetrahydro-pyridazin.

Beispiel 9

6-[5-(1-Imidazolyl)-thien-2-yl]-5-methyl-3-oxo-2,3,4,5-tetrahydro-pyridazin-Fumarsäuresalz.

Eine Mischung aus 200 mg 6-[5-(1-Imidazolyl)-thien-2-yl]-5-methyl-3-oxo-2,3,4,5-tetrahydro-pyridazin und 89 mg Fumarsäure wird in 50 ml Ethanol bis zur Lösung erhitzt. Anschliessend wird eingeengt und der Rückstand getrocknet.

Ausbeute: 214 mg Schmelzpunkt 188–190°C (Zers.)

IR (in KBr): 1708, 1650, 1615 $cm^{-1}$.

Analog Beispiel 9 lassen sich z.B. Oxalate, Succinate, Malonate, Citrate, Tartrate usw. sowie anorganische Salze, wie Hydrochloride, Hydrogensulfate usw., herstellen.

**Patentansprüche für die Vertragsstaaten: BE, CH, DE, FR, GB, IT, LI, LU, NL, SE**

1. Imidazolylalkylthien-2-yl-tetrahydropyridazine der allgemeinen Formel I

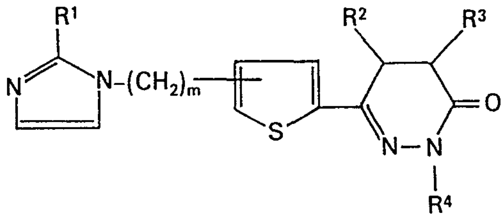

(I)

worin $R^1$, $R^2$, $R^3$ und $R^4$ gleich oder verschieden sein können und unabhängig voneinander ein Wasserstoffatom, einen $C_{1-4}$-Niederalkylrest oder einen Phenylrest bedeuten, m für Null oder eine ganze Zahl von 1–8 steht; ausgenommen sind Verbindungen mit 2,5-disubstituiertem Thiophenrest, bei denen $R^1$, $R^2$, $R^3$ $R^4$ gleichzeitig Wasserstoff bedeuten und m ungleich Null ist.

2. 6-[5-(2-Methyl-1-imidazolyl-methyl)-thien-2-yl]-3-oxo-2,3,4,5-tetrahydro-pyridazin und dessen pharmazeutisch verträgliche Salze.

3. 6-[5-(1-Imidazolyl-methyl)-thien-2-yl]- 2-methyl-3-oxo-2,3,4,5-tetrahydro-pyridazin und dessen pharmazeutisch verträgliche Salze.

4. 6-[5-(1-Imidazolyl-methyl)-thien-2-yl]-3-oxo-2-phenyl-2,3,4,5-tetrahydro-pyridazin und dessen pharmazeutisch verträgliche Salze.

5. 6-[4-(1-Imidazolyl-methyl)-thien-2-yl]-3-oxo-2,3,4,5-tetrahydro-pyridazin und dessen pharmazeutisch verträgliche Salze.

6. 6-[5-(1-Imidazolyl)-thien-2-yl]-5-methyl-3-oxo-2,3,4,5-tetrahydro-pyridazin und dessen pharmazeutisch verträgliche Salze.

7. 6-[5-(1-Imidazolyl)-thien-2-yl]-2,5-dimethyl-3-oxo-2,3,4,5-tetrahydro-pyridazin und dessen pharmazeutisch verträgliche Salze.

8. 6-[5-(2-Methyl-1-imidazolyl)-thien-2-yl]-5-methyl-3-oxo-2,3,4,5-tetrahydro-pyridazin und dessen pharmazeutisch verträgliche Salze.

9. 6-[4-(1-Imidazolyl)-thien-2-yl]-5-methyl-3-oxo-2,3,4,5-tetrahydro-pyridazin und dessen pharmazeutisch verträgliche Salze.

10. Verfahren zur Herstellung von Verbindungen gemäss den Ansprüchen 1–9, dadurch gekennzeichnet, dass man eine 4-[(1-Imidazolyl)-alkyl-thien-2-yl]-4-oxo-buttersäure oder deren Ester der allgemeinen Formel II

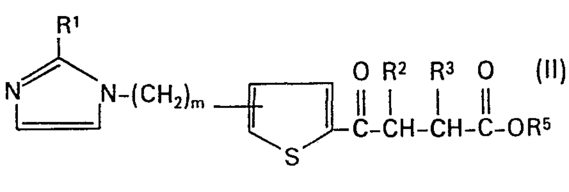

(II)

wobei m, $R^1$, $R^2$ und $R^3$ die in der allgemeinen Formel I angegebenen Bedeutungen haben und $R^5$ Wasserstoff oder $C_{1-6}$-Alkyl bedeutet, mit einer Hydrazinverbindung der allgemeinen Formel III

$H_2N-NH-R^4$ (III)

in der $R^4$ die in der allgemeinen Formel I angegebene Bedeutung hat, deren Hydrat oder Salz in wässrig-alkoholischen oder alkoholischen Medien oder in unter den gewählten Bedingungen indifferenten Lösungsmittel oder deren Mischungen mit Wasser oder Alkohol bei Temperaturen von 30–150°C, gegebenenfalls unter Zuhilfenahme eines der für Aminolysen und Kondensationsreaktionen üblichen Katalysatoren, umsetzt.

**Patentanspruch für den Vertragsstaat: AT**

Verfahren zur Herstellung von Imidazolylalkyl-thien-2-yl-tetrahydropyridazinen der allgemeinen Formel I

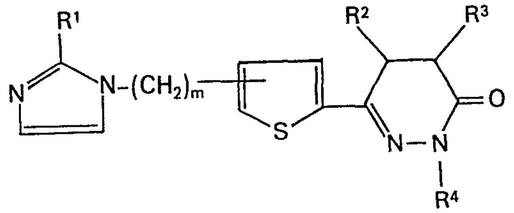

(I)

worin $R^1$, $R^2$, $R^3$ und $R^4$ gleich oder verschieden sein können und unabhängig voneinander ein Wasserstoffatom, einen $C_{1-4}$-Niederalkylrest oder einen Phenylrest bedeuten, m für Null oder eine ganze Zahl von 1–8 steht; ausgenommen sind Verbindungen mit 2,5-disubstituiertem Thiophenrest, bei denen $R^1$, $R^2$, $R^3$, $R^4$ gleichzeitig Wasserstoff bedeuten und m ungleich Null ist, dadurch gekennzeichnet, dass man eine 4-[(1-Imidazolyl)-alkyl-thien-2-yl]-4-oxo-buttersäure oder deren Ester der allgemeinen Formel II

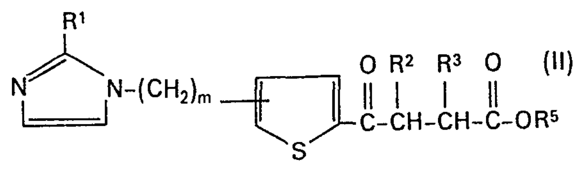

(II)

wobei m, $R^1$, $R^2$ und $R^3$ die in der allgemeinen Formel I angegebenen Bedeutungen haben und $R^5$ Wasserstoff oder $C_{1-6}$-Alkyl bedeutet, mit einer Hydrazinverbindung der allgemeinen Formel III

$H_2-N-NH-R^4$ (III)

in der $R^4$ die in der allgemeinen Formel I angegebene Bedeutung hat, deren Hydrat oder Salz in wässrigen, wässrig-alkoholischen oder alkoholischen Medien oder in unter den gewählten Bedingungen indifferenten Lösungsmitteln oder deren Mischungen mit Wasser oder Alkohol bei Temperaturen von 30–150°C, gegebenenfalls unter Zuhilfenahme eines der für Aminolysen und Kondensationsreaktionen üblichen Katalysatoren, umsetzt.

**Claims for the Contracting States: BE, CH, DE, FR, GB, IT, LI, LU, NL, SE**

1. Imidazolylalkylthien-2-yl-tetrahydropyridazines of the general formula I

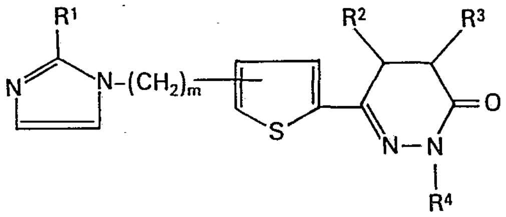

(I)

wherein $R^1$, $R^2$, $R^3$ and $R^4$ can be the same or different and independently of each other denote a hydrogen atom, a $C_{1-4}$ lower alkyl residue or a phenyl residue, m stands for zero or a whole number from 1-8, with the exception of compounds having a 2,5-disubstituted thiophene residue, in which $R^1$, $R^2$, $R^3$ and $R^4$ all denote hydrogen and m is other than zero.

2. 6-[5-(2-Methyl-1-imidazolylmethyl)-thien-2-yl]-3-oxo-2,3,4,5-tetrahydro-pyridazine and its pharmaceutically acceptable salts.

3. 6-[5-(1-Imidazolylmethyl)-thien-2-yl]-2-methyl-3-oxo-2,3,4,5-tetrahydro-pyridazine and its pharmaceutically acceptable salts.

4. 6-[5-(1-Imidazolylmethyl)-thien-2-yl]-3-oxo-2-phenyl-2,3,4,5-tetrahydro-pyridazine and its pharmaceutically acceptable salts.

5. 6-[4-(1-Imidazolylmethyl)-thien-2-yl]-3-oxo-2,3,4,5-tetrahydro-pyridazine and its pharmaceutically acceptable salts.

6. 6-[5-(1-Imidazolyl)-thien-2-yl]-5-methyl-3-oxo-2,3,4,5-tetrahydro-pyridazine and its pharmaceutically acceptable salts.

7. 6-[5-(1-Imidazolyl)-thien-2-yl]-2,5-dimethyl-3-oxo-2,3,4,5-tetrahydro-pyridazine and its pharmaceutically acceptable salts.

8. 6-[5-(2-Methyl-1-imidazolyl)-thien-2-yl]-5-methyl-3-oxo-2,3,4,5-tetrahydro-pyridazine and its pharmaceutically acceptable salts.

9. 6-[4-(1-Imidazolyl)-thien-2-yl]-5-methyl-3-oxo-2,3,4,5-tetrahydro-pyridazine and its pharmaceutically acceptable salts.

10. Process for the preparation of compounds according to claims 1 to 9, characterised in that a 4-[(1-Imidazolyl)-alkyl-thien-2-yl]-4-oxobutyric acid, or an ester thereof of the general formula II

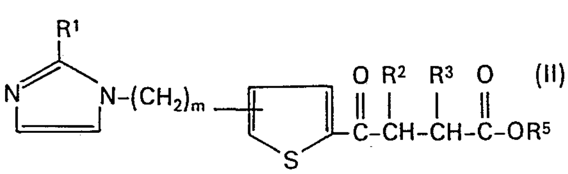

(II)

in which m, $R^1$, $R^2$ and $R^3$ have the meanings given for general formula I and $R^5$ denotes hydrogen or $C_{1-6}$-alkyl, is reacted with a hydrazine compounds of the general formula III

$H_2N\text{-}NH\text{-}R^4$ (III)

in which $R^4$ has the meaning given for general formula I, or with a hydrate or salt thereof, in aqueous, aqueous/alcoholic or alcoholic media or in solvents which are inert in the chosen conditions, or in mixtures thereof with water or alcohol, at temperatures from 30 to 150°C, if necessary with the aid of a catalyst commonly used for aminolyses and condensation reactions.

**Claim for the Contracting State: AT**

Process for the preparation of imidazolylalkylthien-2-yl-tetrahydropyridazines of the general formula I

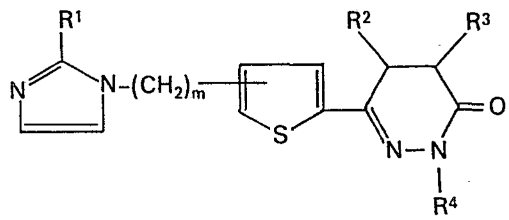

(I)

wherein $R^1$, $R^2$, $R^3$ and $R^4$ can be the same or different and independently of each other denote a hydrogen atom, a $C_{1-4}$ lower alkyl residue or a phenyl residue, m stands for zero or a whole number from 1-8, with the exception of compounds having a 2,5-disubstituted thiophene residue, in which $R^1$, $R^2$, $R^3$ and $R^4$ all denote hydrogen and m is other than zero, characterised in that a 4-[(1-Imidazolyl)-alkyl-thien-2-yl]-4-oxobutyric acid, or an ester thereof, of the general formula II

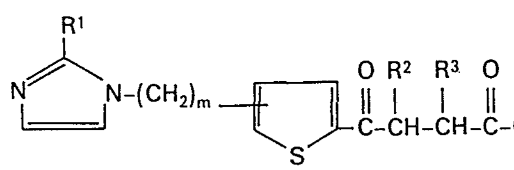

(II)

in which m, $R^1$, $R^2$ and $R^3$ have the meanings given for general formula I and $R^5$ denotes hydrogen or $C_{1-6}$-alkyl, is reacted with a hydrazine compound of the general formula III

$H_2N\text{-}NH\text{-}R^4$ (III)

in which $R^4$ has the meaning given for general formula I, or with a hydrate or salt thereof, in aqueous, aqueous/alcoholic or alcoholic media or in solvents which are inert in the chosen conditions, or in mixtures thereof with water or alcohol, at temperatures from 30 to 150°C, if necessary with the aid of a catalyst commonly used for aminolyses and condensation reactions.

**Revendications pour les Etats contractants: BE, CH, DE, FR, GB, IT, LI, LU, NL, SE**

1. Imidazolylalcoylthién-2-yl-tétrahydropyridazines de formule générale I

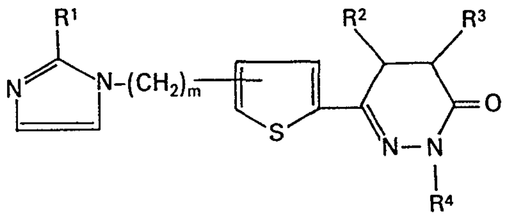

(I)

où $R^1$, $R^2$, $R^3$ et $R^4$ peuvent être identiques ou différents et représentent indépendamment l'un de l'autre un atome d'hydrogène, un radical alcoyle inférieur en $C_1$ à $C_4$ ou un radical phényle, m vaut zéro ou un nombre entier allant de 1 à 8; en exceptant les composés ayant un radical thiophène disubstitué en 2,5 où $R^1$, $R^2$, $R^3$, $R^4$ représentent simultanément un hydrogène et m est différent de zéro.

2. 6-[5-(2-méthyl-1-imidazolyl-méthyl)-thién-2-yl]-3-oxo-2,3,4,5-tétrahydro-pyridazine et ses sels pharmaceutiquement acceptables.

3. 6-[5-(1-imidazolyl-méthyl)-thién-2-yl]-2-méthyl-3-oxo-2,3,4,5-tétrahydro-pyridazine et ses sels pharmaceutiquement acceptables.

4. 6-[5-(1-imidazolyl-méthyl)-thién-2-yl]-3-oxo-2-phényl-2,3,4,5-tétrahydro-pyridazine et ses sels pharmaceutiquement acceptables.

5. 6-[4-(1-imidazolyl-méthyl)-thién-2-yl]-3-oxo-2,3,4,5-tétrahydro-pyridazine et ses sels pharmaceutiquement acceptables.

6. 6-[5-(1-imidazolyl)-thién-2-yl]-5-méthyl-3-oxo-2,3,4,5-tétrahydro-pyridazine et ses sels pharmaceutiquement acceptables.

7. 6-[5-(1-imidazolyl)-thién-2-yl]-2,5-diméthyl-3-oxo-2,3,4,5-tétrahydro-pyridazine et ses sels pharmaceutiquement acceptables.

8. 6-[5-(2-méthyl-1-imidazolyl)-thién-2-yl]-5-méthyl-3-oxo-2,3,4,5-tétrahydro-pyridazine et ses sels pharmaceutiquement acceptables.

9. 6-[4-(1-imidazolyl)-thién-2-yl]-5-méthyl-3-oxo-2,3,4,5-tétrahydro-pyridazine et ses sels pharmaceutiquement acceptables.

10. Procédé de préparation de composés selon les revendications 1–9, caractérisé en ce que on fait réagir un acide 4-[(1-imidazolyl)-alcoyl-thién-2-yl]-4-oxo-butyrique ou ses esters de formule générale II

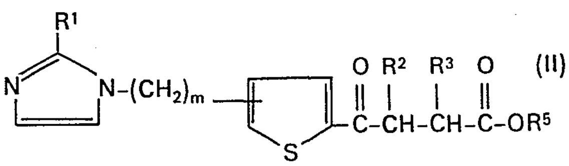

(II)

où m, $R^1$, $R^2$ et $R^3$ ont les significations données dans la formule générale I et $R^5$ représente un hydrogène ou un alcoyle en $C_1$ à $C_6$, avec un composé d'hydrazine de formule générale III

$H_2N\text{-}NH\text{-}R^4$ (III)

où $R^4$ a la signification donnée dans la formule générale I, son hydrate ou sel dans des milieux aqueux, alcooliques-aqueux ou alcooliques dans des solvants indifférents dans les conditions choisies ou leurs mélanges avec de l'eau ou de l'alcool à des températures de 30–150°C, le cas échéant à l'aide de l'un des catalyseurs habituels pour les aminolyses et les réactions de condensation.

**Revendication pour l'Etat contractant: AT**

Procédé de préparation de imidazolylalcoyl-thién-2-yl-tétrahydropyridazines de formule générale I

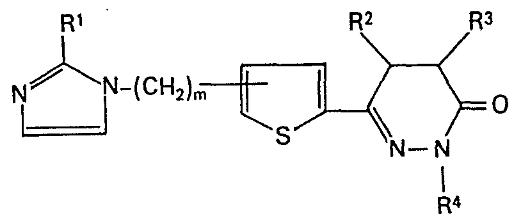

(I)

où $R^1$, $R^2$, $R^3$ et $R^4$ peuvent être identiques ou différents et représentent indépendamment l'un de l'autre un atome d'hydrogène, un radical alcoyle inférieur en $C_1$ à $C_4$ ou un radical phényle, m vaut zéro ou un nombre entier allant de 1 à 8; en exceptant les composés ayant un radical thiophène disubstitué en 2,5 où $R^1$, $R^2$, $R^3$ et $R^4$ représentent simultanément un hydrogène et m est différent de zéro, caractérisé en ce qu'on fait réagir un acide 4-[(1-imidazolyl)-alcoyl-thién-2-yl]-4-oxo-butyrique ou un de ses esters de formule générale II

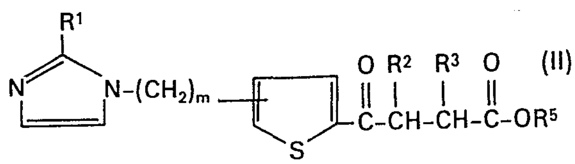

(II)

où m, $R^1$, $R^2$ et $R^3$ ont les significations données dans la formule générale I et $R^5$ représente un hydrogène ou un alcoyle en $C_1$ à $C_6$, avec un composé d'hydrazine de formule générale III

$H_2N\text{-}NH\text{-}R^4$ (III)

où $R^4$ a la signification donnée dans la formule générale I, son hydrate ou sel dans des milieux aqueux, alcooliques-aqueux ou alcooliques ou dans des solvants indifférents dans les conditions choisies ou leur mélange avec l'eau ou l'alcool à des températures de 30–150°C, le cas échéant à l'aide d'un des catalyseurs habituels pour les aminolyses et les réactions de condensation.